# EUROPEAN PATENT APPLICATION

(11) **EP 1 780 547 A1**
(43) Date of publication of application: **02.05.2007**
(21) Application number: 07000742.2
(22) Date of filing: 10.01.2003
(51) Int. Cl.: G01N 33/569

(54) **Methods and devices for collecting and preparing specimens for detection of mycobacteria and their antigens**

(30) Priority: 10.01.2002 US 41667
(62) Divisional of application: 03000366.9
(71) Applicant: Becton, Dickinson and Company, Franklin Lakes, NJ 07417-1880 (US)
(72) Inventor: Siddiqi, Salman H., Sparks Maryland 21152 (US)
(74) Representative: von Kreisler Selting Werner

(57) **Abstract**

Methods are disclosed for detecting a secreted mycobacterial antigen directly in a biological sample obtained from a mammalian subject without substantial replication of mycobacterial cells in the sample. This method comprises contacting an antibody that specifically binds to the secreted mycobacterial antigen with the sample, or a fraction thereof, under conditions such that the antibody specifically binds to secreted mycobacterial antigen in the sample, thereby forming a specific complex of the antibody with the secreted mycobacterial antigen. This method further comprises detecting the presence, absence or amount of specific complex of the antibody with the secreted mycobacterial antigen in the sample. In this method the presence of this specific complex indicates the presence of the secreted mycobacterial antigen in the sample. The invention method is particularly useful for detecting MPB64 antigen of *M. bovis* or MPT64 antigen of *M. tuberculosis* in biological samples from a human subject suspected of having tuberculosis, particularly for sputum samples from such subjects. Prior to contacting the sample with an antibody that specifically binds to MPB64 antigen or MPT64 antigen, a mucolytic or liquifying agent, preferably containing a disulfide bond reducing agent, and optionally a decontaminating agent, is added to the sample. One preferred disulfide bond reducing agent is N-acetyl-L-cysteine (NALC) and advantageously this agent is added to the sample to achieve a concentration in the sample that is substantially greater than 0.25% (w/v), for instance, about 2.5% (w/v).

## Description

### BACKGROUND OF THE INVENTION

### Field of the Invention

The present invention relates generally to methods and articles of manufacture for processing biological samples for rapid and simple detection of mycobacteria and secreted mycobacterial antigens, particularly MPB64 antigen of *Mycobacterium bovis* or MPT64 antigen of *M. tuberculosis.*

### Background of the Technology

Organisms of the genus Mycobacterium are responsible for significant mortality and morbidity in humans. The genus comprises several species which include, but are not limited to, *Mycobacterium africanum, M. avium, M. bovis, M. bovis-BCG, M. chelonae, M. fortuitum, M. gordonae, M. intracellulare, M. kansasii, M. microti, M. scrofulaceum, M. paratuberculosis* and *M. tuberculosis.* Certain of these organisms are the causative agents of disease. Of particular concern is a recent rise in the incidence of tuberculosis (TB), for which the etiological agent is *M. tuberculosis.* However, infections caused by Mycobacteria other than tuberculosis (MOTT) have also been increasing. Many of these new cases are related to the AIDS epidemic, which provides an immune compromised population which is particularly susceptible to infection by Mycobacteria. *M. avium, M. kansasii* and other non-tuberculosis mycobacteria have been found as opportunistic pathogens in HIV-infected and other immune compromised patients. There is therefore an increasing need for rapid differential diagnosis of TB infections, as they can disseminate rapidly and may be fatal within a short period of time.

Traditional methods for detection, isolation and identification of Mycobacterium species include acid-fast staining procedures, culture techniques and biochemical confirmatory procedures. In the first instance, the potential presence of mycobacteria is determined through microscopic examination of a specimen that has been treated with an appropriate acid-fast stain. The presence of acid-fast bacilli (AFB) is indicative of the presence of mycobacteria. For instance, a finding of acid-fast bacilli in sputa or other suitable clinical specimens is considered presumptive evidence of active tuberculosis sufficient to initiate therapy.

After initial visual detection of mycobacteria, a specimen may be further processed and cultured. After growth is attained, further differential tests may be applied to identify the mycobacterial species present. The Center for Disease Control has recommended a series of 18 tests that allow for specific identification of nearly 90 percent of mycobacterial species. Kubica, G. P., in The Mycobacteria: A Sourcebook, Part A (Kubica & Wayne, eds.), pp. 133-175, Marcel Dekkar (New York: 1984).

Diagnosis of mycobacterial infections by cultivation of the organism followed by identification using biochemical assays is time-consuming, and a typical diagnosis using conventional culture methods can take as long as six to eight weeks. Automated culturing systems such as the BACTEC® (Becton Dickinson Microbiology Systems, Sparks, Md.) system can decrease the time for diagnosis to one to two weeks. However, there is still a need to reduce the time required for diagnosing mycobacterial infections to less than a week, preferably to about one day or less. Oligonucleotide probe based assays such as Southern hybridizations or dot blots are capable of returning a rapid result (i.e., in one day or less). Genus- and species-specific primers may also be used in direct assays of clinical samples by nucleic acid amplification. Thus, modem technologies such as BACTEC®, polymerase chain reaction (PCR), and DNA hybridization provide powerful tools for the rapid diagnosis of tuberculosis, but the costs are not easily borne in those countries where tuberculosis is still a life-threatening problem.

Genetic relationships of various mycobacteria have been studied in efforts to prevent and diagnose mycobacterial diseases. For instance, Bacillus Calmette-Guerin (BCG) is the name given to a family of tuberculosis vaccines derived in 1921 by the *in vitro* attenuation of *Mycobacterium bovis.* Subsequently, BCG seed lots were distributed globally, and both phenotypic and genotypic differences between strains have been described. *See, e.g.,* Behr, M. A., et al., A historical and molecular phylogeny of BCG strains, Vaccine 17:915-922 (1999). These authors reviewed the English and French historical record on 13 strains, and DNA was extracted from these strains for restriction-fragment-length-polymorphism study. Molecular typing resulted-in 3 clades, based on variability in IS6110 insertion sequence typing and the presence of one particular gene, the *mpt64* gene. With two exceptions, these clades correspond with strains obtained from the Pasteur Institute in 1924-26 (IS6110-2/*mpt64+*)*,* 1926-31 (IS6110-1/*mpt64+*), and 1931 or later (IS6110-1/*mpt64-*).

Immunological methods for detection of mycobacteria, particularly *M*. *tuberculosis,* are known. For instance, Drowart A. et al., Detection of mycobacterial antigens present in short-term culture media using particle counting immunoassay, Am Rev Respir Dis 147:1401-1406 (1993), discloses a particle counting immunoassay (PACIA) that was compared with the BACTEC® system for detecting mycobacterial growth after short-term culture and was used to identify *M. tuberculosis.* Latex particles were coated with polyclonal anti-BCG or with specific 2A1-2 monoclonal antibody said to specifically recognize a 37-38 kDa culture filtrate protein. Bottles containing nonradioactive Middlebrook 7H9 liquid medium and BACTEC® 12B vials were inoculated with equal amounts of mycobacteria from four reference strains *(M. tuberculosis, M. kansasii, M. avium,* and *M. xenopi).* Using anti-BCG, PACIA detected mycobacterial antigens 3 to 6 days before the BACTEC® system. *M. tuberculosis* was differentiated from the other mycobacteria using 2A1-2. Seventeen clinical samples were also studied. In the same 10, the two techniques detected mycobacteria, PACIA with anti-BCG after 9 days and BACTEC® 1 to 5 days later. For 9 of the 10 samples, PACIA with 2A1-2 detected *M. tuberculosis* after 20 days, a result confirmed with the AccuProbe® (GenProbe, San Diego, CA) system. The authors concluded that PACIA detects mycobacterial growth earlier than BACTEC® and that *M. tuberculosis* can be distinguished from other mycobacteria in PACIA performed with specific monoclonal antibodies.

Another mycobacterial culture filtrate protein that has been characterized immunologically is designated MPB64 or MPT64 in *M. bovis* or *M. tuberculosis,* respectively. Thus, Yamaguchi, R. et al., Cloning and characterization of the gene for immunogenic protein MPB64 of Mycobacterium bovis BCG, Infect. Immun. 57:283-288 (1989), discloses cloning and sequencing of a gene for the MPB64 protein that is found in culture filtrates of only *M. tuberculosis* and some strains of *M. bovis* BCG. The gene was expressed in *Escherichia coli,* and the product showed migration by electrophoresis similar to (authentic) native MPB64 protein and also reacted with polyclonal and monoclonal antibodies raised against authentic protein.

Immunological properties of the MPB64 protein have been further studied using additional recombinant production systems. For example, Oettinger, T. et al., Cloning and B-cell-epitope mapping of MPT64 from Mycobacterium tuberculosis H37Rv., Infect. Immun. 62:2058-64 (1994), discloses cloning and sequencing of the gene *(mpt64)* of the immunogenic protein MPT64 found in unheated culture filtrates of *M. tuberculosis* H37Rv. A comparison showed *mpt64* and the gene encoding the 23-kD MPB64 protein secreted from *M. bovis* BCG Tokyo to be identical except for one silent mutation. Southern blot experiments on genomic mycobacterial DNA showed the presence of MPT64 in the *M. tuberculosis* substrains H37Rv, H37Ra, and Erdman, and MPB64 in the *M. bovis* BCG substrains Tokyo, Moreau, and Russian, whereas the *M. bovis* BCG substrains Glaxo, Pasteur, Canadian, Tice, and Danish 1331 and *Mycobacterium leprae* lack the gene. N-terminal and C-terminal deletion mutants were constructed for mapping of B-cell epitopes on MPT64 with five monoclonal antibodies, C24b1, C24b2, C24b3, L24b4, and L24b5. Western blot (immunoblot) analysis revealed that the murine antibodies bind to one linear and three conformational epitopes.

Oettinger, T. et al., Mapping of the delayed-type hypersensitivity-inducing epitope of secreted protein MPT64 from Mycobacterium tuberculosis, Infect. Immun. 63:4613-8 (1995), discloses that the gene encoding MPT64 *of M. tuberculosis* H37Rv was expressed in *E. coli* K-12 and purified as a recombinant protein. The purified recombinant MPT64 elicited delayed-type hypersensitivity (DTH) in outbred guinea pigs sensitized with *M. bovis* BCG Tokyo. No skin reactions were observed when either recombinant MPT64 or native MPT64 was used in guinea pigs sensitized with *M. bovis* BCG Danish 1331. Studies with overlapping synthetic peptides pinpointed the biological activity at a single DTH-inducing epitope consisting of 15 residues between amino acids Gly-173 and Ala-187. Screening by PCR of 56 clinical isolates of *M. tuberculosis* from Danish and Tanzanian patients demonstrated the presence *of mpt64* in all of the strains. The authors stated that these results point to MPT64 as a possible candidate for a skin test reagent specific for diagnosis of human tuberculosis.

Tasaka, H. et al., Secretion of MPB64 antigen by a recombinant clone of Mycobacterium smegmatis: characterization and application for the diagnosis of tuberculosis, Scand. J. Immunol. 42:487-92 (1995), discloses that MPB64 was produced and secreted by a clone of *M. smegmatis-MPB64* in which the structural gene of MPB64 was inserted using a new vector. Antibodies against the recombinant MPB64 (rMPB64) were used for a reverse particle latex agglutination (RPLA) test to detect the MPB64 antigen rapidly. RPLA tests were applied to stock cultures and clinical isolates of mycobacteria to identify TB complex. In particular, mycobacteria from sputa were isolated on Ogawa egg media, subcultured on Middlebrook 7H9 medium for one week, and then used for the RPLA test. RPLA with anti-MPB64 antibody-coated latex beads completely distinguished TB complex from other mycobacteria. Using polyclonal rabbit anti-rMPB64 antibody-coated latex beads, RPLA detected 1 ng/ml of MPB64, as little as 10³ *M. tuberculosis* in 25 µl of culture medium. The authors suggested that RPLA with anti-MPB64 antibody would be a new, easy and inexpensive method for rapid diagnosis of tuberculosis.

Roche, P. W. et al., Expression of Mycobacterium tuberculosis MPT64 in recombinant Myco. smegmatis: purification, immunogenicity and application to skin tests for tuberculosis, Clin. Exp. Immunol. 103:226-32 (1996a), discloses development of a recombinant mycobacterial vector which secretes the encoded *M*. *tuberculosis* protein MPT64 at high levels into the culture filtrate, from which the protein was isolated by a single-step affinity chromatographic procedure. The purified protein was recognized by both polyclonal and monoclonal anti-MPT64 antibodies. The T cell reactivity of the protein was confirmed by its ability to stimulate human anti-rMPB64 T cell lines. The *M. smegmatis* recombinant MPT64 protein was superior to the *E. coli* rMPB64 protein, which has identical amino acid sequence, in eliciting cutaneous DTH reactions in guinea pigs sensitized with *M. tuberculosis.* The potential of this form of the *M. tuberculosis* MPT64 protein as a skin test reagent for tuberculosis is discussed.

Roche, P. W. et al., Human T-cell epitopes on the Mycobacterium tuberculosis secreted protein MPT64, Scand. J. Immunol. 43:662-70 (1996b), discloses mapping of the immunogenic regions of the MPT64 protein of *M*. *tuberculosis* using peripheral blood mononuclear cells (PBMC) from TB patients and a set of overlapping peptides encompassing the complete sequence of the protein. A minority of TB patients (6/32) mounted an antibody response to MPT64. Sera from half (3/6) of these identified two linear antibody binding sites.

The gene encoding the MPB64 antigen has also been used for detection of mycobacteria in clinical samples via nucleic acid analyses. For instance, Dar, L. et al., Diagnosis of pulmonary tuberculosis by polymerase chain reaction for MPB64 gene: an evaluation in a blind study, Indian. J. Chest. Dis. Allied Sci. 40:5-16 (1998), discloses an evaluation of PCR for the *mpb64* gene in which 182 clinical samples (sputum, bronchioalveolar lavage and pleural fluid) from patients with a clinical diagnosis of pulmonary tuberculosis and 72 samples from patients with non-tubercular pulmonary lesions and normal healthy individuals were included. PCR was positive in 59% of single sputum samples from clinically diagnosed pulmonary tuberculosis, while *M. tuberculosis* could be grown in 18% of the samples. PCR could identify *M. tuberculosis* in 81.8% of the culture positive sputum samples. PCR was also positive in 71.4% of bronchioalveolar lavage (BAL) fluid and 60.7% pleural fluid samples from clinically suspected cases, which were mostly culture negative. The authors stated that "PCR for MPB64 gene provides a useful alternative for the diagnosis of pulmonary tuberculosis from sputum and paucibacillary samples like BAL and pleural fluid in which conventional methods show low sensitivity, especially in areas from which strains show a low copy number of other PCR targets like the IS6110 insertion sequence" (Abstract).

Japanese patent JP11108931 to Nanba, et al. discloses a method for identification of *M. tuberculosis* in a culture medium using an anti-MPB64 antibody. More recently, Abe, C. et al., "Simple and rapid identification of the Mycobacterium tuberculosis complex by immunochromatographic assay using anti-MPB64 monoclonal antibodies", J. Clin. Microbiol. 37:3693-3697 (1999), discloses that an immunochromatographic assay (MPB64-ICA) for identification of the *M. tuberculosis* complex was evaluated with 20 reference strains of mycobacterial species and 111 clinical isolates. MPB64-ICA displayed a very strong reaction band with organisms belonging to the *M. tuberculosis* complex but not with mycobacteria other than *M. tuberculosis* (MOTT bacilli), except for one of four *M. marinum* strains tested and one *M. flavescens* strain, both of which gave very weak signals. The effectiveness of MPB64-ICA was tested in combination with two liquid culture systems (MB-REDOX, Biotest AG, Dreieich, Germany; and the Mycobacteria Growth Indicator Tube (MGIT®) system; BBL Becton Dickinson Microbiology Systems, Cockeysville, Md.). A total of 108 of 362 sputum specimens processed were positive for acid-fast bacilli. Samples taken from the cultures on the same days when either of the two culture systems became positive for mycobacteria were assayed with MPB64-ICA. Of 108 cultures with mycobacteria, 51 showed a positive signal with the test, in which the presence of the *M. tuberculosis* complex was demonstrated later by the AccuProbe® nucleic acid assay for *M. tuberculosis* complex. In addition, MPB64-ICA correctly detected the *M. tuberculosis* complex in mixed cultures of the *M. tuberculosis* complex and MOTT bacilli. According to the authors, these results indicate that MPB64-ICA can be easily used for rapid identification of the *M. tuberculosis* complex in combination with culture systems based on liquid media without any technical complexity in clinical laboratories. The authors noted, however, that "[w]hen fresh *M. bovis* BCG grown in a liquid medium was used for the evaluation, the analytical sensitivity of MPB64-ICA was calculated as 10⁵ CFU/ml (Table 3), a value similar to that for the AccuProbe test [citations omitted]. Therefore, this test should not be used directly on fresh clinical specimens" (p. 3996, c. 1).

U. S. Patent No. 6,245,331 to Laal et al. discloses that a number of protein and glycoprotein antigens secreted by *Mycobacterium tuberculosis* (Mtb) have been identified as "early" Mt antigens on the basis of early antibodies present in subjects infected with Mt prior to the development of detectable clinical disease. These early Mt antigens, in particular an 88 kDa secreted protein having a pI of about 5.2 present in Mt lipoarabinomannan-free culture filtrate, a protein characterized as Mt antigen 85C; a protein characterized as Mt antigen MPT51, a glycoprotein characterized as Mt antigen MPT32; and a 49 kDa protein having a pI of about 5.1, are useful in immunoassay methods for early, rapid detection of TB in a subj ect.

Processing of biological (e.g., sputum) for detection of mycobacteria is generally directed first at liquification and removal of cellular or other debris and reduction of contaminating microorganisms, rather than at purification of mycobacteria per se. Thus, methods of processing biological and inorganic samples suspected of containing one or more mycobacteria, for the detection of such mycobacteria, are constrained by the presence of other, undesired microorganisms naturally present in such samples. When attempts are made to culture the slow-growing mycobacteria from the processed samples, however, often breakthrough growth of other faster-growing organisms present in the processed sample could overtake the culture, thereby causing a potential false positive result. Such breakthrough growth is a problem as it can hinder or prevent the detection of slow-growing bacteria, and especially mycobacteria, in the sample. This problem is especially a concern for the culture of slow-growing pathogenic species of mycobacteria, as a patient can be misdiagnosed if the suspected microorganism cannot be cultured from that patient's samples. Hence, a need exists for improved methods for promoting the selective recovery of a desired microorganism, especially pathogenic mycobacteria, from a sample that may contain many other faster-growing microorganisms.

More in particular, to prepare for *M. tuberculosis* culture, sputum samples often are still processed today by methods which have been in use for about forty years (G. Kubica, et al., Am. Rev. Resp. Dis. 87:775-779 (1963)). These and other sample processing methods for *M. tuberculosis* culture have been developed for the purposes of 1) reducing the viscosity of the sputum sample to facilitate handling, 2) killing contaminating organisms (such as fungi and non-mycobacteria) to prevent co-culturing with *M. tuberculosis* and confusion of the diagnosis, and 3) concentrating the sample into a small volume for seeding cultures. To accomplish these goals, conventional sample preparation methods for Mycobacteria sp. have traditionally employed harsh conditions and caustic, toxic or reactive reagents. Conventional sample processing procedures for mycobacterial culture are reviewed, for instance, by G. Kubica in The Mycobacteria : A Source Book. Part A. (Kubica, G. and Wayne, L. eds.) Marcel Dekker, N.Y. (1984), pp. 315-344.

U. S. Patent No. 5,364,763 to Kacian discloses methods and compositions for improved liquification of mucoid secretions by treatment with a disulfide bond reducing agent and a DNA digestion agent, and for improved concentration of selected bacterial species from such specimens. In addition Kacian provides an historical overview of the development of various processing methods for clinical samples to be tested for various microbiological organisms, particularly mycobacteria, as follows. According to Kacian, investigators in the 1950's to early 1960's recognized that there were differences in the composition of various mucoid secretions, including sputa, depending upon source and the nature of a given disease process. In particular, the existence of sputa that were classified as mucoid and lacked inflammatory cells such as are commonly found in association with infectious processes, as well as sputa containing such cells (classified as purulent), was appreciated. It was suggested that both protein components and DNA were likely to contribute to sputum viscosity, and evidence in support of this was collected. In particular, DNAses and proteases were shown to have the ability to liquify sputa and pus to some extent. DNAses appeared to have more activity when used with purulent sputa; whereas, proteases were more effective with mucoid sputa.

Further according to Kacian, in addition to enzymes, a variety of other agents such as detergents, salts, oxidizing and reducing agents have been tried as liquifying agents, and a number worked more or less successfully. In the early 1960's, the use of the sulfhydryl reagent N-acetyl-L-cysteine ("NALC") was reported effective for liquifying mucoid secretions, both purulent and mucoid in nature. Webb, J., Thoracic & Cardiovascular Surg., 44:330-343 (1962). It was claimed that the reagent was capable of liquifying both mucous (the protein component thought to be responsible for most of the viscosity in mucoid specimens) and DNA, which had been postulated to be a significant contributor to viscosity in purulent specimens. This reagent and the related compound, dithiothreitol (disclosed in U.S. Pat. No. 3,502,779 ("the '779 patent"), issued to Dye et al. in 1974), gave superior liquification of many mucoid specimens for a variety of purposes, as judged by comparison with previous procedures.

More in particular, Dye et al. exemplifies the use of dithiothreitol and related compounds for liquification of mucus in clinical specimens using dithiothreitol in 0.5% solution with 2% sodium hydroxide and 0.1 M sodium citrate, using as a control the same solution containing 0.5 % N-acetyl-L-cysteine (c. 2,11. 31-65). In some of the tests, the concentration of the dithiothreitol was reduced to 0.15%, and it was observed that sputum liquefication was as good with that as with the N-acetyl-L-cysteine at 0.5%. There were more positive smears with the dithiothreitol reagent in accordance with the invention and in general, the number and size of colonies were greater. The mycobacteria isolated included drug-susceptible and multiple drug-resistant tubercle bacilli, *M. kansasii,* and Battey-type bacilli. Dye et al. also notes that dithiothreitol is susceptible to fairly rapid oxidation in dilute aqueous solution, so that when so used, the solution should be made up fresh at or about the time of use; or made up in de-aerated water and promptly sealed in ampoules and the like with the usual precautions being taken to prevent access of oxygen.

U. S. Patent No. 5,554,503 to Down et al. discloses methods for processing samples which may contain mycobacteria which are compatible with both conventional culturing techniques and nucleic acid analysis. According to the method of Down et al., a sample, usually a clinical sample such as sputum, is first processed by conventional procedures for liquification and decontamination (i.e., killing of non-mycobacteria) such as the NALC procedure described above. In conventional culturing methods, the NALC pellet is resuspended in 1.5-2 ml of an appropriate buffer or water for seeding solid or liquid growth media. To prepare the sample for nucleic acid analysis, the pellet is washed to remove inhibitory, caustic and toxic reagents which may interfere with the subsequent nucleic acid analysis, and then heat treated to release nucleic acids.

In addition to difficulties relating to liquification of sputum and other mucoid or viscous specimens, isolation or concentration of bacterial species from such specimens has been problematic in certain cases. In particular, bacteria of the genus Mycobacterium are difficult to separate in satisfactory quantities from sputum specimens because their density is close to that of water, which renders centrifugation inefficient. The aforementioned U. S. Patent No. 5,364,763 to Kacian also discloses improved methods for concentrating selected bacterial species from mucosal secretion specimens or other biological specimens for the purposes of assaying for the presence of such species. In particular, white blood cells associated with bacteria are concentrated, preferably by centrifugation, and then are selectively lysed such that the bacteria remain substantially cellularly intact. The concentrated bacteria, for example mycobacteria, may then be assayed in the concentrated specimen using disclosed methods and compositions. More recently, U. S. Patent No. 6,126,839 to Kreader et al. discloses methods for concentrating bacteria from a viscous biological sample. The methods involve adding to the sample a water-soluble, density-lowering agent having a density of 0.7 to 0.9 g/ml and a boiling point greater than 50° C.

To control contamination of clinical specimens with non-mycobacterial organisms, it is known that the culture broth can be provided with an antibacterial and antifungal/antibiotic cocktail such as that sold under the trade name "PANTA" made by Becton Dickinson. This product will reduce possible contamination by bacteria such as *Pseudomonas aeruginosa* or *Candida tropicalis.* For instance, Whittier, S. et al., Improved recovery of mycobacteria from respiratory secretions of patients with cystic fibrosis, J. Clin. Microbiol. 31:861-864 (1993), discloses that decontamination of respiratory specimens (n = 121) with 0.25% N-acetyl-L-cysteine and 1 % sodium hydroxide (NALC-NaOH) was associated with a high rate of pseudomonas overgrowth for both Lowenstein-Jensen slants (74%) and BACTEC® 12B vials supplemented with PANTA (polymyxin B [50 U/ml], amphotericin B [5 micrograms/ml], nalidixic acid [20 micrograms/ml], trimethoprim [5 micrograms/ml], azlocillin [10 micrograms/ml]) (36%). This overgrowth limited recovery of mycobacteria to only 64% (9 of 14) of specimens positive by smear for acid-fast bacilli (AFB). Decontamination of specimens (n = 441) with NALC-NaOH, followed by 5% oxalic acid treatment, resulted in contamination of only 5% of Lowenstein-Jensen slants and 3% of BACTEC® vials. AFB were recovered from all 90 AFB smear-positive specimens following the use of this decontamination technique.

U. S. Patent No. 5,985,593 to Thornton et al. discloses methods for the enzymatic decontamination of specimens that are said to be especially useful to eliminate non-gram negative contaminants of samples being processed for microbiological analysis. The patent discloses that lytic enzyme treatment can, if desired, be provided prior to subsequent handling of the specimen or sample, for example, in the field or location at which the specimen or sample is first taken, and the specimen or sample can then be frozen or otherwise stabilized against decomposition until further processing is desired or can be performed. The patent also states that, alternatively, the container into which the sample is placed can be coated with or otherwise contain a desired lytic enzyme composition. Transport of samples, especially solid samples, in a solution containing the lytic enzyme compositions of the invention, is said to be especially useful to protect against further contamination of the sample or against the growth of endogenous contaminants already present on the sample. For instance, this patent teaches that the lytic enzymes can be premixed with the NALC solution which is used in the NALC liquefaction step for processing a clinical specimen.

### SUMMARY OF THE INVENTION

It is an object of the present invention is to provide a simple and rapid method of detecting mycobacteria and secreted mycobacterial antigens, particularly an MPB64 antigen of *M. bovis* or an MPT64 antigen of *M. tuberculosis,* in biological samples.

It is another object of this invention to provide a method for detecting infection of a mammalian subject by a mycobacterial species that produces a secreted mycobacterial antigen, particularly an MPB64 antigen or an MPT64 antigen, by testing a biological sample from the subject for the presence of either of these antigens without substantial replication of mycobacterial cells in the sample, particularly without time consuming replication by conventional mycobacterial culturing methods.

Yet another object of this invention is to provide articles of manufacture, such as kit comprising a container for sample collection and processing and related reagents, to facilitate detecting infection of a mammalian subject by a mycobacterial species that produces MPB64 antigen or MPT64 antigen by testing a biological sample from the subject for these antigens without substantial replication of mycobacterial cells in the sample.

Still another object of the present invention is to provide a self-contained device for sample collection, transport, processing and testing, such as a container containing all reagents needed for liquifying and decontaminating a biological sample, such as a sputum specimen, as well as for readily detecting MPB64 antigen or MPT64 antigen in such a sample, such as by a color indicator, simply by introducing the biological sample into the container and subjecting the container to a few simple manipulations.

These and other objects are provided by the present invention which is based in part upon the finding that a secreted mycobacterial antigen, particularly an MPB64 antigen or an MPT64 antigen, can be detected in biological samples from mammals infected with a mycobacterial species that secretes such an antigen, particularly clinical specimens such as sputum from human subjects infected with *M. tuberculosis,* without substantial replication of mycobacterial cells in the sample before antigen detection. The invention also provides methods and articles of manufacture for improving recovery and sensitivity of detection of mycobacteria and secreted mycobacterial antigens, particularly MPB64 antigen or MPT64 antigen, in a biological sample.

One aspect of the present invention, therefore relates to a method for detecting a secreted mycobacterial antigen in a biological sample obtained from a mammalian subject without substantial replication of mycobacterial cells in the sample. This method comprises contacting an antibody that specifically binds to the secreted mycobacterial antigen with the sample, or a fraction thereof, under conditions such that the antibody specifically binds to secreted mycobacterial antigen in the sample, thereby forming a specific complex of the antibody with the secreted mycobacterial antigen. This method further comprises detecting the presence, absence or amount of specific complex of the antibody with the secreted mycobacterial antigen in the sample. In this method the presence of this specific complex indicates the presence of the secreted mycobacterial antigen in the sample.

In contrast to prior methods for determining whether a biological sample contains a secreted mycobacterial antigen, which require culturing of mycobacterial cells in the sample for a week or longer prior to testing for antigen, the invention method provides for detecting a secreted mycobacterial antigen in a biological sample even when, after the biological sample is obtained from the subject until detection of the antigen, mycobacterial cells in the sample do not replicate substantially. In other words, although some insubstantial replication of mycobacterial cells in a sample may incidentally occur after sample collection, prior to testing for antigen, during transport or storage of the sample, for instance, substantial replication of mycobacterial cells in the test sample, such as by deliberate culturing of the cells in mycobacterial culture media, is not required for detecting a secreted mycobacterial antigen in a biological sample according to the present invention.

The invention method is particularly useful for detecting MPB64 antigen or MPT64 antigen in biological samples from a human subject suspected of having tuberculosis, particularly for sputum samples from such subjects. However, the invention method also may be applied to other mucoid secretion specimens or other bodily fluids or materials.

In one embodiment of this method, prior to contacting the sample with an antibody that specifically binds to MPB64 antigen or MPT64 antigen, a mucolytic or liquifying agent is added to sample, preferably a liquifying agent that comprises a disulfide bond reducing agent. One preferred disulfide bond reducing agent is N-acetyl-L-cysteine and sufficient N-acetyl-L-cysteine (NALC), and advantageously this agent is added to the sample to achieve a concentration in the sample that is substantially greater than 0.25% (w/v), a concentration used in conventional sputum processing methods (see, e.g., G. Kubica in The Mycobacteria: A Source Book. Part A. (Kubica, G. and Wayne, L. eds.) Marcel Dekker, N.Y. (1984), pp. 315-344). Preferably, N-acetyl-L-cysteine is added to the sample to achieve a concentration in the sample of about 2.5% (w/v).

In the method of detection of a secreted mycobacterial antigen, advantageously the antibody that specifically binds to the secreted mycobacterial antigen is affixed to a solid matrix. Preferably, detecting the presence, absence or amount of the specific complex of that antibody with the secreted mycobacterial antigen comprises using a reagent that produces a color that indicates the presence of specific complex on the solid matrix. In one embodiment using an antibody on a solid matrix, prior to contacting the sample with the antibody, the sample is treated to separate mycobacterial cells from the remainder of the sample, thereby providing a mycobacterial cell fraction and a remainder fraction of the sample. The mycobacterial cell fraction is then tested for the presence of mycobacterial cells, either visually or by conventional culturing methods. The antibody that specifically binds to the secreted mycobacterial antigen is then contacted with the remainder fraction of the sample.

In a preferred embodiment using antibody affixed to a solid matrix and fractionation of the sample, the mycobacterial cell fraction is prepared by passing the sample through a filter that retains mycobacterial cells and does not retain the secreted antigen, for instance, MPB64 antigen or MPT64 antigen, whereby the resulting retentate on the filter becomes the mycobacterial cell fraction and the resulting filtrate becomes the remainder fraction. The solid matrix carrying the antibody may then also comprise a filter, for instance, a membrane filter, in a filter stack comprising the filter that retains mycobacterial cells and this membrane filter, fluidly connected in series. The filter stack may be situated within a container such that a sample introduced into the container flows first through the filter that retains mycobacterial cells and then through the membrane filter carrying the antibody.

Another aspect the present invention relates to a method for detecting an infection of a mammalian subject by a mycobacterial species that produces a secreted mycobacterial antigen by testing a biological sample obtained from that subject for the secreted mycobacterial antigen without substantial replication of mycobacterial cells in the sample. This method comprises contacting an antibody that specifically binds to the secreted mycobacterial antigen with the sample or a fraction thereof under conditions such that the antibody specifically binds to the secreted mycobacterial antigen in the sample or fraction thereof, thereby forming a specific complex of the antibody with the secreted mycobacterial antigen. This method further comprises detecting the presence, absence or amount of the specific complex of the antibody with the secreted mycobacterial antigen in the sample or fraction thereof, where the presence of the specific complex indicates infection of the subject by the mycobacterial species. In this method, after the sample is obtained from the subject until the step of detecting the antigen-antibody complex is completed, mycobacterial cells in the sample do not replicate substantially. In preferred embodiments of this method, the secreted mycobacterial antigen is an MPB64 antigen or an MPT64 antigen.

In another aspect, this invention relates to a method of processing a biological sample obtained from a mammalian subject for detecting a secreted mycobacterial antigen, such as MPB64 antigen or MPT64 antigen, in the sample. This method comprises adding a liquification and, optionally, a decontamination agent, to the sample prior to the step of detecting the antigen, and after the sample is obtained from the subject until the detecting of antigen is completed, maintaining the sample under conditions such that mycobacterial cells in the sample do not replicate substantially. Preferably, the sample is maintained under conditions such that contaminating bacterial cells also do not replicate substantially, for instance, due to addition of a decontamination agent. In this method advantageously the liquifying agent comprises N-acetyl-L-cysteine in an amount sufficient to achieve a concentration in the sample that is substantially greater than 0.25% (w/v). By substantially greater than 0.25% (w/v) is meant at least about two times, preferably about four times and more preferably about eight times that level. Still more preferably, the liquifying agent comprises N-acetyl-L-cysteine in an amount sufficient to achieve a concentration in the sample of about 2.5% (w/v). The liquifying agent also may advantageously comprise a nuclease, particularly a DNase that is capable of cleaving DNA in the biological sample, as disclosed, for instance, in U. S. Patent No. 5,364,763 to Kacian.

In this sample processing method of the invention, preferably the sample is maintained at a substantially neutral pH after collection until after detecting the presence, absence or amount of antibody that is specifically bound to secreted mycobacterial antigen, such as MPB64 antigen or MPT64 antigen, in the sample. Thus, the present inventor has found that addition to a sputum sample of NaOH, for instance, at 1-2% (w/v) as in conventional "NaOH-NALC" liquification procedures, substantially reduces reactivity of MPB64 antigen or MPT64 antigen with antibodies that specifically bind to these antigens. Treatment with such conventional concentrations of NaOH also reduces viability of mycobacteria in a biological sample, thereby reducing the sensitivity of subsequent tests for mycobacterial antigen or viable cells detected by culture. By "a substantially neutral pH" is meant, for instance, a pH between about 4 and about 10, preferably between about 5 and about 9, more preferably between about 6 and about 8, and most preferably in the range of about 6.5 to about 7.5.

The invention method for processing a biological sample for detecting a secreted mycobacterial antigen optionally further comprises adding a decontamination agent to the sample prior to detecting a secreted antigen. For instance, the decontaminating agent may comprise a mixture of antibiotics, such as the mixture known as "PANTA" which comprises polymyxin B, amphotericin B, nalidixic acid, trimethoprim and azlocillin in amounts sufficient to achieve concentrations in the sample of at least about 50 U/ml, 5 micrograms/ml, 20 micrograms/ml, 5 micrograms/ml and 10 micrograms/ml, respectively. More preferably, the decontaminating agent comprises polymyxin B, amphotericin B, nalidixic acid, trimethoprim and azlocillin in amounts sufficient to achieve concentrations in the sample of about 500 U/ml, 50 micrograms/ml, 200 micrograms/ml, 50 micrograms/ml and 100 micrograms/ml, respectively. The decontaminating agent also may comprise other antibiotics or enzymes that selectively kill or inhibit replication of non-mycobacterial cells.

Yet another aspect of the present invention relates to an article of manufacture comprising a container, a liquification agent and packaging material containing the container and the liquification agent. The packaging material includes a label that indicates that the container is to be used for combining the liquification agent with a biological sample obtained from a mammalian subject for detecting a secreted mycobacterial antigen, such as an MPB64 antigen or an MPT64 antigen, in the sample or a fraction thereof, without substantial replication of mycobacterial cells in the sample. Advantageously, the liquification agent in this article of manufacture is N-acetyl-L-cysteine in a solid form. This solid form optionally further comprises a decontamination agent (e.g., PANTA). Preferably, the liquification agent and, optinonally, the decontamination agent, is contained in the container of this article of manufacture in solid form, so that a biological sample introduced into the container contacts the liquification agent. For instance, the solid liquification agent may be in the form of a coating on a surface inside of the container.

Preferably, the container of the invention further comprises a first solid support and a second solid support, where the first solid support retains mycobacterial cells and does not retain a secreted mycobacterial antigen, such as MPB64 antigen or MPT64 antigen, and the second solid support has affixed to it a capture antibody that specifically binds to that secreted antigen. In this embodiment, the first solid support and the second solid support are disposed inside the container such that a biological sample introduced into the container contacts the first solid support and the second solid support. The first solid support advantageously comprises a first porous matrix and the second solid support comprises a second porous matrix, where the first and second porous matrices are disposed so that when a biological sample is introduced into the container, the biological sample passes though the first porous matrix, whereby mycobacterial cells in the sample are retained by the first porous matrix, and thereafter the biological sample passes through the second porous matrix, whereby the secreted mycobacterial antigen, such as MPB64 antigen or MPT64 antigen in the sample is specifically bound to the capture antibody on the second porous matrix.

Advantageously, in this embodiment of the article of manufacture of the invention, the first porous matrix is removable from the container after the biological sample passes through it, such that mycobacterial cells retained by the first porous matrix are removed from the container on that matrix. This removable first porous matrix therefore provides a convenient method for concentrating mycobacterial cells in a sample for further testing, such as staining and visual inspection or conventional culturing tests.

Advantageously, this article of manufacture of the invention further comprises a detection reagent that specifically detects secreted mycobacterial antigen, such as MPB64 antigen or MPT64 antigen, that is specifically bound to the capture antibody, and thereby generates a detectable signal on the second porous matrix.

In a preferred embodiment of the article of manufacture of this invention, the container contains all reagents needed for detecting a secreted mycobacterial antigen, such as MPB64 antigen or MPT64 antigen, in a biological sample. These reagents include one that produces a color that indicates the presence of antigen specifically bound to the capture antibody on the second porous matrix. In this embodiment, the label indicates that the container contains all reagents needed for detecting the secreted mycobacterial antigen in a biological sample that is introduced into the container.

### BRIEF DESCRIPTION OF THE DRAWINGS

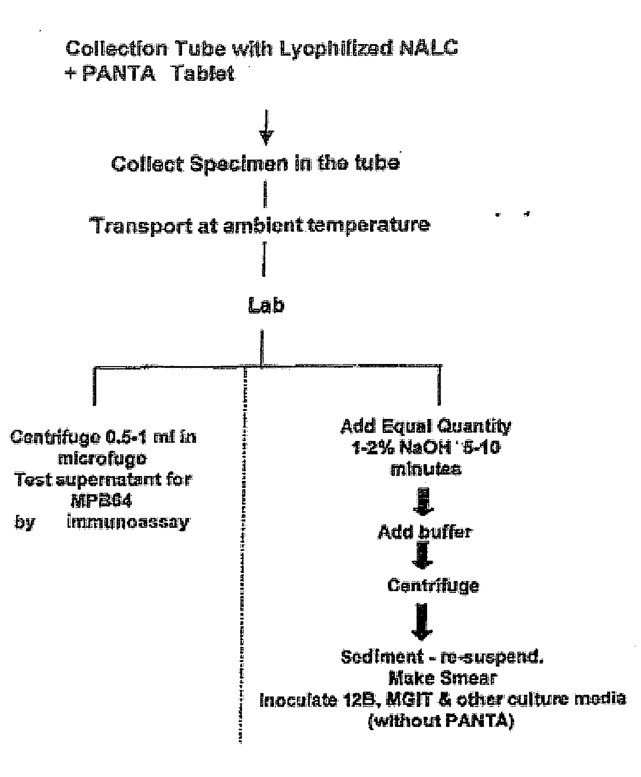

Figure 1 shows a flow diagram of a preferred embodiment of the invention method for detecting a secreted mycobacterial antigen in a biological sample obtained from a mammalian subject without substantial replication of mycobacterial cells in the sample. In this method, a tablet containing a lyophilized liquification agent (NALC) and decontamination agent (PANTA) is placed in the sample collection tube prior to collection of the specimen (e.g., sputum), and the sample is transported to the site of mycobacterial antigen assay at ambient temperature. There the sample is fractionated by centrifugation, the supernatant is tested directly for antigen, and the pellet is optionally treated with NaOH and tested for acid-fast bacilli (AFB) and viable mycobacteria by conventional culture methods.

### DETAILED DESCRIPTION OF THE INVENTION

The present invention is based in part upon the finding that a secreted mycobacterial antigen, particularly an MPB64 antigen or an MPT64 antigen, can be detected in biological samples from mammals infected with a mycobacterial species that secretes such an antigen, particularly clinical specimens such as sputum from human subjects infected with *M. tuberculosis,* without substantial replication of mycobacterial cells in the sample before antigen detection.

The following definitions and explanations of terms apply throughout the present disclosure unless otherwise indicated.

One convention in mycobacterial protein nomenclature is the use of MPB and MPT numbers. MPB denotes a protein purified from *M. bovis* BCG followed by a number denoting its relative mobility in 7.7% polyacrylamide gels at a pH of 9.5. MPT denotes a protein isolated from *M. tuberculosis. See, e.g.,* U. S. Patent No. 6,245,331 to Laal et al.

The term "secreted mycobacterial antigen" as used herein includes any member of the family of at least four secreted proteins with common structural features that is produced and secreted by mycobacteria, and can be found in the fluid surround the cells, as described hereinbelow, including antigens displaying an epitope of an 85A, 85B, 85C, MPT51 or MPT64 or MPB64 secreted protein.

Wiker and colleagues have studied a family of secreted Mt proteins which include a complex of 3 proteins termed antigens 85A, 85B and 85C (also known as the "85 complex" or "85cx"). *Se*e, e.g., Wiker, H. G. et al., Scand. J. Immunol. 36:307-319 (1992); Wiker, H. G. et al., Microbiol. Rev. 56:648-661 (1992). This complex was originally found in *M. bovis* BCG preparations which produced a secreted antigen comprising a complex of three closely related components, antigen 85A, 85B, and 85C (Wiker, H. G. et al., Int. Arch. Allergy Appl. Immunol. 81:289-306 (1986)). The corresponding components of Mt are also actively secreted. The 85 complex is considered the major secreted protein constituent of mycobacterial culture fluids though it is also found in association with the bacterial surface. In most SDS-polyacrylamide gel electrophoresis (SDS-PAGE) gels, 85A and C are not resolved, whereas isoelectric focusing resolves three distinct bands.

Genes encoding six of the secreted proteins: 85A, 85B, 85C, "antigen 78" (usually referred to as the 38 kDa protein), MPB64 and MPB70 have been cloned. Three separate genes located at separate sites in the mycobacterial genome encode 85A, B and C (Content, J. et al., Infect. Imnun. 59:3205-3212(1991)). A gene encoding the antigen known as MPT-32 (reported as a 45/47 kDa secreted antigen complex) has been cloned, sequenced and expressed (Laqueyrerie, A. et al., Infec. Immun. 63:4003-4010 (1995)) and designated as the apa gene. The antigen 85 complex is often referred to as the "30/31 kDa doublet," although slightly different molecular mass designations have been reported.

The following list shows the molecular masses of the individual components of antigen 85 complex plus two additional antigens (in SDS-PAGE) as described by Wiker and colleagues, along with alternative nomenclatures:
Ag85A = MPT44 = 31 kDa
Ag85B = MPT59 = 30 kDa
Ag85C = MPT45 = 31.5 kDa
   MPT64 = about 26 kDa
   MPT51 = about 27 kDa
Ag78 -- = 38 kDa
   MPT32 = 45/47 kDa (38/42 kDa by Laal et al., *supra).*

Wiker's group studied cross-reactions between five actively secreted Mt proteins by crossed immunoelectrophoresis, SDS-PAGE with immunoblotting and enzyme immunoassay (EIA) using (1) polyclonal rabbit antisera to the purified proteins and (2) a mouse monoclonal antibody ("mAb"). The mAb HBT4 reacted with the MPT51 protein. The 85A, 85B, and 85C constituents cross-reacted extensively, though each had component-specific in addition to cross-reacting epitopes. These components also cross-reacted with MPT51 and MPT64. Amino acid sequence homology was shown between 85A, 85B, 85C and MPT51. MPT64 showed less homology. Striking homology was also found between two different structures within the 85B sequence. Thus a family of at least four secreted proteins with common structural features has been demonstrated in mycobacteria.

The phrase "without substantial replication" as used, for instance, in describing a method for detecting a secreted mycobacterial antigen in a biological sample obtained from a mammalian subject without substantial replication of mycobacterial cells in the sample, is used to indicate that the subject cells have not been subjected to conditions conducive to extensive replication, such as conventional mycobacterial culturing conditions. In contrast to prior methods for determining whether a biological sample contains a secreted mycobacterial antigen, particularly methods for detecting MPT64 antigen disclosed by Abe et al., *supra,* which require culturing of mycobacterial cells in the sample for a week or longer prior to testing for antigen, the invention method provides for detecting a secreted mycobacterial antigen in a biological sample even when, after the biological sample is obtained from the subject until detection of the antigen, mycobacterial cells in the sample do not replicate substantially.

In other words, although some insubstantial replication of mycobacterial cells in a sample may incidentally occur after sample collection, prior to testing for antigen, during transport or storage of the sample, for instance, substantial replication of mycobacterial cells in the test sample, such as by deliberate culturing of the cells in mycobacterial culture media, is not required for detecting a secreted mycobacterial antigen in a biological sample according to the present invention. Accordingly, "without substantial replication" in the present context indicates that mycobacterial cells in a sample are maintained without at least a 100 fold increase in the original number of mycobacterial cells in the sample, preferably without a ten fold increase, and more preferably even without a three fold increase.

By "antibody" is meant any peptide or peptide mimetic that binds to an antigen in an immunologically specific manner. Typically, this includes but is not limited to intact polyclonal and monoclonal antibodies and derivatives including antigen binding fragments, including such immunoglobulin fragments as Fab, F(ab')₂, Fab', scFv (both monomers and polymeric forms) and isolated H and L chains. An antigen binding fragment retains the ability to specifically bind to an antigen , although avidity and/or affinity may be altered compared, for instance, to a related antibody.

By "antibiotic" is meant a compound that has a deleterious effect on the viability, integrity, or competence of a contaminant, as understood in the art. Examples of different classes of antibiotics include the β-lactam antibiotics, the β-lactamase inhibitors, the aminoglycosides and aminocyclitols, the quinolones, tetracyclines, macrolides, and lincosamides, as well as the glycopeptides, lipopeptides and polypeptides, the sulfonamides and trimethoprim, chloramphenicol, isoniazid, nitroimidazoles, rifampins, nitrofurans, methenamine, and mupirocin. Other antifungal compounds include the polyenes, azoles, and pyrimidine synthesis inhibitors. Any or all of these antibiotics are useful in conjunction with the methods of the invention. The term antibiotic is synonymous with "antimicrobial" as used herein.

By "biological sample" is meant a sample derived from, or taken from, a specimen of biological origin, such as a specimen taken from an animal (including human) or plant. Biological samples can be derived from, or taken from any part of the biological organism, including but are not limited to expectorated matter (for example, sputum, saliva and phlegm), bronchial lavages and analogous respiratory washings, feces, tissue samples including skin samples, gastric aspirates, urine, tears, perspiration, blood and cerebral spinal fluid (CFS). Any animal species may be used as a source for such samples, including but not limited to ruminant animals (such as members of the bovine family (cattle, cows, etc.) or members of the ovine family (sheep, etc.)), pigs, fish and members of the avian family.

By "inorganic sample" is meant a sample derived from, or taken from, a non-biological specimen, such as, for example, from an environmental source such as soil, mud, sludge, water, sawdust and air.

One aspect of the present invention relates to a method for detecting a secreted mycobacterial antigen in a biological sample obtained from a mammalian subject without substantial replication of mycobacterial cells in the sample. This method comprises contacting an antibody that specifically binds to the secreted mycobacterial antigen with the sample, or a fraction thereof, under conditions such that the antibody specifically binds to secreted mycobacterial antigen in the sample, thereby forming a specific complex of the antibody with the secreted mycobacterial antigen. The method further comprises detecting the presence, absence or amount of specific complex of the antibody with the secreted mycobacterial antigen in the sample. In this method the presence of this specific complex indicates the presence of the secreted mycobacterial antigen in the sample.

Detection of a specific complex of an antibody with a secreted mycobacterial antigen may be performed using any immunoassay format of which many are known in the art. Thus, polyclonal or monoclonal antibodies may be used in immunoassay protocols of several types. The antibodies may be used intact or fragments may be generated which are also capable of binding to the target antigen. While immunoassays can be performed using only polyclonal antibody reagents, in most cases monoclonal antibody or a combination of polyclonal and monoclonal antibodies are preferred.

In general, antibodies or antigens in immunoassays are labeled by conjugation to a detectable label to facilitate detection of antigen/antibody binding by inclusion of the label in the binding complexes formed. As used herein, the term "detectable label" and related terms are intended to encompass both the detectable label alone and, as described below, detectable labels associated with particles. Suitable detectable labels and methods for conjugating them to proteins such as antibodies are well known. Directly detectable labels, which do not require additional reagents or reaction to be detected, include radioisotopes, fluorescent dyes and visible absorbing dyes. Enzymes capable of reacting to produce colored products are suitable indirectly detectable labels commonly used for conjugation to antibodies in specific binding assays. All of the foregoing detectable labels are suitable for conjugation to polyclonal and monoclonal antibodies for use in antigen detection steps of methods of the present invention.

In the method of detecting a secreted mycobacterial antigen of the invention, advantageously the antibody that specifically binds to the secreted mycobacterial antigen is affixed to a solid matrix. Preferably, detecting the presence, absence or amount of the specific complex of that antibody with the secreted mycobacterial antigen comprises using a reagent comprising a detectable label that indicates the presence of the specific complex on the solid matrix. For instance, the specific complex of antibody and secreted mycobacterial antigen may be dectected with a detectably labeled second antibody hat specifically binds to the same antigen as the first antibody that is affixed to a solid support, but at a different site (epitope). Preferably, the detectable label on this second antibody comprises a reagent that exhibits a color that is readily detectable by the naked eye.

Protocols for immunoassays are well known in the art. For example, polyclonal or monoclonal antibodies or antigen binding fragments thereof may be employed in sandwich assays for detecting the secreted mycobacterial antigen, or in known modifications and variations of sandwich assay protocols. Alternatively, antibodies and antigen binding fragments thereof may be employed in various competitive assay formats as are known in the art. For detection of MPB64 or MPT64 antigen, one particularly preferred immunoassay is an immunochromatographic assay using anti-MPB64 monoclonal antibodies, as described by Abe, C. et al., *supra.* One commercially available form of this immunoassay is available, the "Capilia TB Slide Test", a lateral flow immunochromatographic assay from Tauns (Namazo City, Japan). Western blot (immunoblot) analysis can also be used for detection of MPB64 or MPT64 antigen, as these antigens are known to exhibit at least one linear epitope. *See, e.g.,* Oettinger, T. et al., 1994, *supra.*

The invention method is particularly useful for detecting MPB64 antigen or MPT64 antigen in biological samples from a human subject suspected of having tuberculosis, particularly for sputum samples from such subjects. However, the invention method also may be applied to other mucoid secretion specimens of, for example, cervical mucous, bronchial mucous, vaginal mucous or other mucous taken from a human or other animal. The invention can also be applied to other biological samples or specimens such as urine, cerebral spinal fluid, whole blood, or other body fluids and secretions.

In one embodiment of the invention method, prior to contacting a biological sample with an antibody that specifically binds to MPB64 antigen or MPT64 antigen, a mucolytic or liquifying agent is added to sample, preferably a liquifying agent that comprises a disulfide bond reducing agent. One preferred disulfide bond reducing agent is N-acetyl-L-cysteine (NALC) and sufficient N-acetyl-L-cysteine (NALC), and advantageously this agent is added to the sample to achieve a concentration in the sample that is substantially greater than 0.25% (w/v), a concentration used in conventional sputum processing methods *(see, e.g.,* G. Kubica in The Mycobacteria: A Source Book. Part A. (Kubica, G. and Wayne, L. eds.) Marcel Dekker, N.Y. (1984), pp. 315-344). Preferably, N-acetyl-L-cysteine is added to the sample to achieve a concentration in the sample of about 2.5% (w/v). NALC is preferred because it is compatible with culture tests, including automated culturing instruments such as the BACTEC® instruments that are commercially available from Becton, Dickinson Diagnostic Instrument Systems, Sparks, Md.

Other disulfide bond reducing agents are useful in conjunction with the methods of the invention if they act in a manner similar to NALC. Examples of such reducing agents include, but are not be limited to, dithiothreitol (DTT) and related compounds disclosed in U.S. Pat. No. 3,502,779 to Dye et al., and β-mercaptoethanol (β-ME).

In one embodiment of the mycobacterial antigen detection method, the sample is treated to separate mycobacterial cells from the remainder of the sample, thereby providing a mycobacterial cell fraction and a remainder fraction containing the antigen. The mycobacterial cell fraction is then tested for the presence of mycobacterial cells, for instance, visually (e.g., by acid-fast staining) or by conventional culture methods. The antibody that specifically binds to the secreted mycobacterial antigen is then contacted with the remainder fraction of the sample.

In a preferred embodiment using such fractionation of the sample and further using an antibody affixed to a solid matrix, the mycobacterial cell fraction is prepared by passing the sample through a filter that retains mycobacterial cells and does not retain the secreted antigen, for instance, MPB64 antigen or MPT64 antigen, whereby the resulting retentate on the filter becomes the mycobacterial cell fraction and the resulting filtrate becomes the remainder fraction containing the antigen. The solid matrix carrying the specific antibody may then also comprise a filter, for instance, a membrane filter, in a filter stack comprising the filter that retains mycobacterial cells and this membrane filter, fluidly connected in series. The filter stack may be situated within a container such that a sample introduced into the container flows first through the filter that retains mycobacterial cells and then through the membrane filter carrying the antibody.

Another aspect the present invention relates to a method for detecting an infection of a mammalian subject by a mycobacterial species that produces a secreted mycobacterial antigen by testing a biological sample obtained from that subject for the secreted mycobacterial antigen without substantial replication of mycobacterial cells in the sample. This method comprises contacting an antibody that specifically binds to the secreted mycobacterial antigen with the sample or a fraction thereof under conditions such that the antibody specifically binds to the secreted mycobacterial antigen in the sample or fraction thereof, and detecting the presence, absence or amount of antibody bound to the antigen, as described above.

In another aspect, this invention relates to a method of processing a biological sample obtained from a mammalian subject for detecting an antigen, particularly a secreted mycobacterial antigen such as MPB64 antigen or MPT64 antigen, as well as bacterial cells, particularly mycobacterial cells, in the sample. This method comprises adding a liquification agent to the sample at the time of sample collection or otherwise prior to the step of detecting the antigen, and after the sample is obtained from the subject until the detection of antigen is completed, maintaining the sample under conditions such that bacterial cells in the sample do not replicate substantially. In this method advantageously the liquifying agent comprises N-acetyl-L-cysteine in an amount sufficient to achieve a concentration in the sample that is substantially greater than the conventional level of 0.25% (w/v). Preferably, the liquifying agent comprises N-acetyl-L-cysteine in an amount sufficient to achieve a concentration in the sample of about 2.5% (w/v). The liquifying agent also may advantageously comprise a nuclease, particularly a DNase that is capable of cleaving DNA in the biological sample, as disclosed, for instance, in U. S. Patent No. 5,364,763 to Kacian. In this sample processing method of the invention, preferably the sample is maintained at a substantially neutral pH after collection until after detecting the target antigen, and more preferably, until after detecting bacterial cells. In other words, conventional decontamination treatments with strong base, e.g., 1-2% NaOH, are preferably avoided throughout sample processing to avoid attendant reduction in viability of mycobacteria in a biological sample, thereby reducing the sensitivity of subsequent tests for mycobacterial antigen or viable cells.

Examples of pathogens that can be extracted from samples prepared according to the invention and that are capable of causing diseases and conditions of heightened importance in testing include especially the causative agents of tuberculosis *(M. tuberculosis* complex) and leprosy *(M. leprae* (human leprosy) and *M. lepraemurium* (rodent leprosy)); *Mycobacterium avium* complex bacteria (important bird pathogens); *M. avium* (sometimes isolated from AIDS patients who are afflicted with a mycobacterial superinfection); *M bovis* (of importance in veterinary medicine); *M. fortuitum* (a soil bacterium that has been isolated from lesions in animals and humans); *M. intracellulare* (opportunistic and especially seen in patients infected with the AIDS virus); *M. paratuberculosis* (of interest in the diagnosis of Crohn's disease (regional ileitis) in humans); *Mycobacterium kansasii* (a rare but devastating agent, generally associated with pulmonary disease); *Mycobacterium marinum* (infects cold-blooded animals and fish and has also been isolated from superficial granulomas on the extremities of humans); *Mycobacterium paratuberculosis* (the causative agent of Johne's disease in cattle; it is very slow growing and cultures must be held for 16 weeks before it can be assured that they are negative); and *M. ulcerans* (also of interest in human medicine).

Detecting organisms containing mycolic acid structures in their outer membranes in samples prepared by the method of the invention can be done using techniques such as culture, nucleic acids amplification, and immunodiagnostics.

When it is desired to culture the mycobacteria from the sample, methods known in the art may be used. Examples of liquid media useful for mycobacterial culture include BACTEC® 12B (also called "12B") (Becton-Dickinson, Cockeysville, MD), ESP® MYCO System II (DIFCO Laboratories, Detroit, MI) or MB/BacT® (Organon Teknika, Durham, NC). Examples of non-selective solid media useful for the culture of mycobacteria include Lowenstein-Jensen (L-J), 7H10 or 7H11. Examples of selective solid media useful for the culture of mycobacteria include L-J Gruft, Mycobactosel or 7H11 1-selective. Whereas the selective solid media is generally preferred, relative to the non-selective media, the liquid culture media, which is generally more sensitive, is preferred with the methods of the invention. Commercial preparations of antibiotic supplements for use in conjunction with the aforementioned liquid culture systems include PANTA (Becton-Dickinson, Cockeysville, MD), PVNA (DIFCO Laboratories, Detroit, MI), and MAS (Organon Teknika, Durham, NC), respectively. Each of these culture systems can be modified to incorporate different or additional antibiotics to optimize diagnostic performance in conjunction with the methods of the invention. For example, modification of the PANTA formulation to include vancomycin and/or ceftazidime is known in the art.

The invention method for processing a biological sample for detecting a secreted mycobacterial antigen optionally further comprises adding a decontamination agent to the sample prior to detecting a secreted antigen. The decontamination agent serves to selectively kill or inhibit non-mycobacterial cells which might otherwise interfere with subsequent tests, particularly culture tests, for mycobacteria. Addition of the decontamination agent immediately upon sample collection is advantageous, particularly where the sample is to be stored or transported for an extended period, particularly without refrigeration in warm climates. The decontaminating agent my comprise a mixture of antibiotics, such as the mixture known as "PANTA" which comprises polymyxin B, amphotericin B, nalidixic acid, trimethoprim and azlocillin in amounts sufficient to achieve concentrations in the sample of at least about 50 U/ml, 5 micrograms/ml, 20 micrograms/ml, 5 micrograms/ml and 10 micrograms/ml, respectively. More preferably, the decontaminating agent comprises the antibiotics in PANTA at about five to ten times the above level, for instance, polymyxin B, amphotericin B, nalidixic acid, trimethoprim and azlocillin in amounts sufficient to achieve concentrations in the sample of about 500 U/ml, 50 micrograms/ml, 200 micrograms/ml, 50 micrograms/ml and 100 micrograms/ml, respectively. Thus, the present inventor has found that addition of PANTA to biological samples to achieve these latter levels (about ten times higher than conventional use of PANTA) provides greater reduction of non-mycobacterial contaminants without unacceptably reducing recovery of viable mycobacterial cells, particularly *M*. *tuberculosis* cells. Higher concentrations of antimicrobials such as present in PANTA, if added to growth medium, can be shown to be inhibitory to the growth of many mycobacteria.

The decontaminating agent also may comprise other antibiotics or enzymes that selectively kill or inhibit replication of non-mycobacterial cells. For instance, Whittier. S. et al., *supra,* discloses decontamination of respiratory specimens with NALC-NaOH, followed by 5% oxalic acid treatment. Instead of conventional NALC-NaOH treatment, however, the present inventor has found that a higher concentration of NALC in the complete absence of NaOH provides superior recovery of viable mycobacteria. Alternatively, instead of adding the usual concentration of NaOH to a biological sample (up to a concentration of 1-2% (w/v)) at the same time NALC is added, the present inventor has found it advantageous to add NaOH for a shorter time and or at a lower concentration (e.g., about 10 time less or about 0.1% (w/v)) than in conventional NALC-NaOH liquification-decontamination methods. Preferably, NaOH is added only after detection of antigen is completed, if such detection is desired, and then only for a relatively short time, for instance, 10-20 min., before neutralization and, optionally, washing of cells to remove neutralized NaOH.

Alternatively, or in addition, the decontamination of biological samples processed according to the present invention also may comprise use of a lytic enzyme as a decontamination agent, as disclosed, for instance, in U. S. Patent No. 5,985,593 to Thornton et al., which discloses methods for enzymatic decontamination of specimens that eliminate non-gram negative contaminants.

Yet another aspect of the present invention relates to an article of manufacture for processing a biological sample for detecting a secreted mycobacterial antigen. This article of manufacture, for instance, a kit, comprises a container, a liquification agent and packaging material containing the container and the liquification agent. The packaging material includes a label that indicates that the container is to be used for combining the liquification agent with a biological sample obtained from a mammalian subject for detecting a secreted mycobacterial antigen, such as an MPB64 antigen or an MPT64 antigen, in the sample or a fraction thereof, without substantial replication of mycobacterial cells in the sample.

Advantageously, the liquification agent in this article of manufacture comprises a disulfide bond reducing agent, preferably N-acetyl-L-cysteine, in a solid form, for instance, in the form of a powder or microparticles, optionally in a pelletized form, such as a tablet, or in a capsule. This solid form optionally further comprises a decontamination agent, such as an antibiotic or antibiotic mixture, or a lytic enzyme or mixtures thereof. Preferably, the liquification agent is contained in the container of this article of manufacture, so that a biological sample introduced into the container contacts the liquification agent. For instance, the solid liquification agent may be in the form of a coating on a surface inside of the container, or it could be a tablet to be introduced into the specimen collection tube at the time of collection.

Preferably, the container of the invention further comprises a first solid support and a second solid support, where the first solid support retains mycobacterial cells and does not retain a secreted mycobacterial antigen, such as MPB64 antigen or MPT64 antigen, and the second solid support has affixed to it a capture antibody that specifically binds to that secreted antigen. In this embodiment, the first solid support and the second solid support are disposed inside the container such that a biological sample introduced into the container contacts the first solid support and the second solid support. The first and second solid supports advantageously comprise a first and second porous matrix, such as suitable filters or membranes in a filter stack, where the first and second filters or membranes are disposed so that when a biological sample is introduced into the container, the biological sample passes though the first filter, whereby mycobacterial cells in the sample are retained by that filter, and thereafter the biological sample passes through the second filter, whereby the secreted mycobacterial antigen, such as MPB64 antigen or MPT64 antigen in the sample is specifically bound to the capture antibody on the second filter.

Prior to filtration, the sample may optionally be treated with an agent that selectively lyses white blood cells under conditions mycobacteria inside or associate with such cells remain substantially cellularly intact, as disclosed, for instance, in U. S. Patent No. 5,364,763 to Kacian.

Filters that selectively retain bacterial cells, including mycobacterial cells, while allowing proteins such as secreted mycobacterial antigens to pass, including MPB64 and MPT64 antigens, are well known in the art. If desired, additional prefilters or post-filters may be included in the filter stack, for instance, to dispense a reagent (e.g., a liquifying agent) into the stream or to remove material larger than bacterial cells prior to collecting those cells. Alternatively or in addition, prior to filtration, insoluble material larger than mycobacterial cells that might exceed the removal capacity of the filter stack may be removed by other means, for instance, centrifugation or passing the mixture over a Spin-X II column fitted with a 20-60 micron frit (Coming Costar, Boston, Mass.). Such a column might also contain a matrix, such as Sephadex® (G-50: Pharmacia, Piscataway, NJ) or an equivalent resin, to enhance purification. If necessary, the container can be fitted with an aperture suitable for applying vacuum or pressure to facilitate filtration of the liquified sample, or the container may be configured as a centrifuge tube, or in a form that can be attached to or inserted into such a tube, so that centrifugation (by an electric powered centrifuge or by hand) can be applied to facilitate filtration of the liquified sample.

In the sample processing container of the invention, advantageously the first porous matrix (filter) is removable from the container after the biological sample passes through it, such that mycobacterial cells retained by the first porous matrix are removed from the container on that matrix. This removable first porous matrix therefore provides a convenient method for concentrating mycobacterial cells in a sample for further testing, such as staining and visual inspection or conventional culturing tests.

The article of manufacture, such as a kit, advantageously further comprises a detection reagent that specifically detects secreted mycobacterial antigen, such as MPB64 antigen or MPT64 antigen, when that antigen is specifically bound to the capture antibody on the second porous matrix (filter), and thereby generates a detectable signal on the second porous matrix.

In a preferred embodiment, the container contains all reagents needed for detecting a secreted mycobacterial antigen, such as MPB64 antigen or MPT64 antigen, in a biological sample. These reagents include a detection reagent that produces a color that indicates the presence of target antigen specifically bound to the capture antibody on the second porous matrix. The geometry of the second filter can be optimized for visual detection of a minimal deposit of the colored detection reagent. For instance, the surface area of the filter carrying the capture antibody can be minimized so as to concentrate the associated color, and the filter may be disposed in the container so that an edge of the filter it can be viewed without removal of the filter from the container, thereby providing a longer path for transmission of light through the associated color and, hence, increasing the sensitivity of detection of the immunological complex, compared to viewing a larger surface of the filter.

In this embodiment of the article of manufacturer, the label of the packaging material indicates that the container contains all reagents needed for detecting the secreted mycobacterial antigen in a biological sample that is introduced into the container. Sample processing with this container consists of simply adding the biological sample to the container which already contains the necessary liquification agent(s) and, if desired, decontaminating agent(s), and agitating the container to mix the sample with those agents. For instance, a sample of sputum may be collected directly into this container. The sample may then be stored and/or shipped in the container until testing for antigen or cells is desired. The container and filters may be so designed and manipulated that filtration of the liquified sample occurs immediately upon adding the sample or only later, for instance, just before testing for antigen or cells, through further manipulation of the container (e.g., application of vacuum or pressure or centrifugal force). If the antigen test is positive, the entire container may be disposed of without opening it for further testing, thereby minimizing exposure of personnel to viable pathogens.

Alternatively, the aforementioned removability from the container of the first filter, on which mycobacterial cells are collected, facilitates further testing for such cells, by providing a convenient means for concentrating most or all of such cells from the sample onto a solid form that is easily handled. Use of filtration to concentrate mycobacterial cells also overcomes the known problem that bacteria of the genus Mycobacterium are difficult to separate in satisfactory quantities from sputum specimens because their density is close to that of water, which renders centrifugation inefficient.

### Experimental

Direct Testing of Smear-Positive Sputum Specimens for the Detection of MPT64. The commercially available "Capilia TB Slide Test", a lateral flow immunochromatographic assay from Tauns (Namazo City, Japan), was used for the testing.

a. Effect of NaOH and NALC on the test: Preliminary experiments indicated that NaOH at the concentration typically used in conventional NALC-NaOH sample preparation destroys the antigen-antibody reaction. After neutralization, the assay device control reaction that indicates the validity of the test is restored but the test reaction is still affected. In contrast, NALC did not affect the test even at 10X the concentration conventionally used. It was therefore concluded that at least about 2.5% NALC (w/v) without NaOH could be used to digest a clinical specimen for an immunoassay to detect MPT64 antigen.

b. Direct testing of sputum specimens: Eight smear-positive specimens (frozen), positive for acid-fast bacilli (AFB), were tested. Smear-positivity of each sample was rated at 1+ (that is, 1-10 AFB/microscopic field) to 3+ (100 or more AFB/microscopic field). Results and conclusions: The Tauns device was used for the MPT64 antigen test. As shown by results below, when a digested specimen was applied to the devise without centrifugation, much of the specimen did not flow through the membrane. However, when the specimen was centrifuged after digestion with NALC, the sample flowed well. Direct antigen test results for MPT64 antigen, using the Tauns test, were as follows.

| **Specimen #** | **AFB Value** | **Test Results** | **Control Results** |
|---|---|---|---|
| 8310 | > 10/field | + | + |
| 45871 | > 10/field | + | + |
| 8182 | > 10/field | No Flow | No Flow |
| 8182 Centrifuged | | + | + |
| 2751 | > 10/field | + | + |
| 2751 Centrifuged | | + | + |
| 7531 | 1-10/field | No Flow | No Flow |
| 7531 Centrifuged | | + | + |
| 5257 | 1-10/field | No Flow | No Flow |
| 5257 Centrifuged | | + | + |
| 8308 | 1-10/field | +/- | + |
| 7658 | < 1/field | No Flow | No Flow |
| 7658 Centrifuged | | + | + |

| | | | |
|---|---|---|---|
| +/- = pink color but much weaker than the control | | | |

c. Culture results: Two specimens were cultured with and without the addition of PANTA in the medium.

| Detection of Growth (in days) | | |
|---|---|---|
| | 12B¹ (n=2, GI 50) | MGIT® 960² (n=2) |
| 8182 with PANTA³ | 7 | 8.6 |
| 8182 without PANTA | 7 | 8.4 |
| 7658 with PANTA | 11 | 11.9 |
| 7658 without PANTA | 11 | 11.3* |

| | | |
|---|---|---|
| *= One tube contaminated 1. Radiometric BACTEC® 12B medium (Becton Dickinson). During metabolism of a radiolabelled substrate in the medium, CO₂ is liberated and C¹⁴ in the liberated CO₂ is detected by a BACTEC® 460 instrument. 2. Non-radiometric BACTEC® MGIT® 960 medium (Becton Dickinson). Growth is detected by fluorescence of a sensor in the bottom of the tube, which changes due to consumption of oxygen by bacteria during growth. 3. All commercial products were used as directed by the manufacturer (Becton Dickinson). | | |

Based on the above results, the following sample processing approach was developed for routine use in place of conventional NACL-NaOH procedures, as illustrated in Figure 1. A sample to be tested for MPT64 antigen is collected into a tube containing lyophilized NALC (sufficient to achieve 2.5% (w/v) after sample addition) and PANTA, or the lyophilized NALC and PANTA are added to the tube in tablet form, both preferably at a concentration 5 to 10 times higher than generally recommended. The sample is then transported and/or stored at ambient temperature until tested for antigen. Then a small portion (e.g., 0.1-0.5 ml) of the liquified sample is centrifuged, for instance, in a microfuge, and the supernatant is tested for MPT64 antigen by immunoassay. To the remaining liquified sample is added an equal volume of 0.5-1% (w/v) NaOH, and the sample is incubated at room temperature for 5-10 minutes. Neutralization solution (0.068 M sodium and potassium phosphate) is then added, the sample is centrifuged, and the sediment is re-suspended to make a smear and to inoculate standard culture media (optionally, without PANTA).

The invention having now been fully described with reference to representative embodiments and details, it will be apparent to one of ordinary skill in the art that changes and modifications can be made thereto without departing from the spirit or scope of the invention as set forth herein.
1. A method for detecting a secreted mycobacterial antigen in a biological sample obtained from a mammalian subject without substantial replication of mycobacterial cells in said sample, said method comprising:
   contacting an antibody that specifically binds to said secreted ycobacterial antigen with said sample or a fraction thereof under conditions such that said antibody specifically binds to said secreted mycobacterial antigen in said sample or fraction thereof, thereby forming a specific complex of said antibody with said secreted mycobacterial antigen, and
   detecting the presence, absence or amount of said specific complex of said antibody with said secreted mycobacterial antigen in said sample or fraction thereof, the presence of said specific complex indicating the presence of said secreted mycobacterial antigen in said sample,
   wherein after said sample is obtained from said subject until said detecting is completed, mycobacterial cells in said sample do not replicate substantially.
2. The method of item 1 wherein said secreted mycobacterial antigen is an MPB64 antigen or an MPT64 antigen.
3. The method of item 1 wherein
   prior to said contacting, said sample is treated to separate mycobacterial cells from the remainder of said sample, thereby providing a mycobacterial cell fraction and a remainder fraction of said sample,
   said mycobacterial cell fraction is tested for the presence of mycobacterial cells, and
   said antibody that specifically binds to said secreted mycobacterial antigen is affixed to a solid matrix and is contacted with said remainder fraction of said sample.
4. A method for detecting an infection of a mammalian subject by a mycobacterial species that produces a secreted mycobacterial antigen by testing a biological sample obtained from said subject for said secreted mycobacterial antigen without substantial replication of mycobacterial cells in said sample, said method comprising:
   contacting an antibody that specifically binds to said secreted mycobacterial antigen with said sample or a fraction thereof under conditions such that said antibody specifically binds to said secreted mycobacterial antigen in said sample or fraction thereof, thereby forming a specific complex of said antibody with said secreted mycobacterial antigen, and
   detecting the presence, absence or amount of said specific complex of said antibody with said secreted mycobacterial antigen in said sample or fraction thereof, the presence of said specific complex indicating infection of said subject by said mycobacterial species,
   wherein after said sample is obtained from said subject until said detecting is completed, mycobacterial cells in said sample do not replicate substantially.
5. The method of item⁴ wherein said secreted mycobacterial antigen is an MPB64 antigen or an MPT64 antigen.
6. A method of processing a biological sample obtained from a mammalian subject for detecting MPB64 antigen or MPT64 antigen in said sample comprising adding a liquification agent to said sample prior to said detecting, and
   after said sample is obtained from said subject until said detecting is completed, maintaining said sample at a substantially neutral pH and under conditions such that mycobacterial cells in said sample do not replicate substantially.
7. The method of item⁶ wherein said liquifying agent comprises N-acetyl-L-cysteine in an amount sufficient to achieve a concentration in said sample that is about or substantially greater than 0.25% (w/v), or wherein said liquifying agent comprises a DNase that is capable of cleaving DNA in said biological sample.
8. An article of manufacture comprising a container, a liquification agent and packaging material containing said container and said liquification agent,
   wherein said packaging material includes a label that indicates that said container is to be used for combining said liquification agent with a biological sample obtained from a mammalian subject for detecting MPB64 antigen or MPT64 antigen in said sample or a fraction thereof without substantial replication of mycobacterial cells in said sample.
9. The article of manufacture of item⁸ wherein said container further comprises a first solid support and a second solid support, wherein
   said first solid support retains mycobacterial cells and does not retain MPB64 antigen or MPT64 antigen,
   said second solid support has affixed thereto a capture antibody that specifically binds to MPB64 antigen or MPT64 antigen, and
   said first solid support and said second solid support are disposed inside said container such that a biological sample introduced into said container contacts said first solid support and said second solid support.
10. The article of manufacture of item⁹ wherein said first solid support comprises a first porous matrix and said second solid support comprises a second porous matrix, wherein said first porous matrix and said second porous matrix are disposed so that when a biological sample is introduced into said container,
   said biological sample passes though said first porous matrix, whereby mycobacterial cells in said sample are retained by said first porous matrix, and thereafter
   said biological sample passes through said second porous matrix, whereby MPB64 antigen or MPT64 antigen in said sample is specifically bound to said capture antibody on said second porous matrix; and further wherein said first solid matrix is removable from said container after said biological sample passes through said first porous matrix such that mycobacterial cells retained by said first porous matrix are removed from said container on said first porous matrix; and further comprising a detection reagent that specifically binds to said MPB64 antigen or MPT64 antigen that is specifically bound to said capture antibody and thereby generates a detectable signal on said second porous matrix; and further
wherein said container further contains all reagents needed for detecting MPB64 antigen or MPT64 antigen in a biological sample including a reagent that produces a color that indicates the presence of MPB64 antigen or MPT64 antigen specifically bound to said capture antibody on said second porous matrix, and
said label indicates that said container contains all reagents needed for detecting MPB64 antigen or MPT64 antigen in a biological sample that is introduced into said container.

## Claims

1. A method for detecting a secreted mycobacterial antigen in a biological sample obtained from a mammalian subject without substantial replication of mycobacterial cells in said sample, said method comprising:
contacting an antibody that specifically binds to said secreted ycobacterial antigen with said sample or a fraction thereof under conditions such that said antibody specifically binds to said secreted mycobacterial antigen in said sample or fraction thereof, thereby forming a specific complex of said antibody with said secreted mycobacterial antigen, and
detecting the presence, absence or amount of said specific complex of said antibody with said secreted mycobacterial antigen in said sample or fraction thereof, the presence of said specific complex indicating the presence of said secreted mycobacterial antigen in said sample,
wherein after said sample is obtained from said subject until said detecting is completed, mycobacterial cells in said sample do not replicate substantially.

2. The method of Claim 1 wherein said secreted mycobacterial antigen is an MPB64 antigen or an MPT64 antigen.

3. The method of Claim 1 wherein
prior to said contacting, said sample is treated to separate mycobacterial cells from the remainder of said sample, thereby providing a mycobacterial cell fraction and a remainder fraction of said sample,
said mycobacterial cell fraction is tested for the presence of mycobacterial cells, and
said antibody that specifically binds to said secreted mycobacterial antigen is affixed to a solid matrix and is contacted with said remainder fraction of said sample.

4. A method for detecting an infection of a mammalian subject by a mycobacterial species that produces a secreted mycobacterial antigen by testing a biological sample obtained from said subject for said secreted mycobacterial antigen without substantial replication of mycobacterial cells in said sample, said method comprising:
contacting an antibody that specifically binds to said secreted mycobacterial antigen with said sample or a fraction thereof under conditions such that said antibody specifically binds to said secreted mycobacterial antigen in said sample or fraction thereof, thereby forming a specific complex of said antibody with said secreted mycobacterial antigen, and
detecting the presence, absence or amount of said specific complex of said antibody with said secreted mycobacterial antigen in said sample or fraction thereof, the presence of said specific complex indicating infection of said subject by said mycobacterial species,
wherein after said sample is obtained from said subject until said detecting is completed, mycobacterial cells in said sample do not replicate substantially.

5. The method of Claim 4 wherein said secreted mycobacterial antigen is an MPB64 antigen or an MPT64 antigen.

6. A method of processing a biological sample obtained from a mammalian subject for detecting MPB64 antigen or MPT64 antigen in said sample comprising
adding a liquification agent to said sample prior to said detecting, and
after said sample is obtained from said subject until said detecting is completed, maintaining said sample at a substantially neutral pH and under conditions such that mycobacterial cells in said sample do not replicate substantially.

7. The method of Claim 6 wherein said liquifying agent comprises N-acetyl-L-cysteine in an amount sufficient to achieve a concentration in said sample that is about or substantially greater than 0.25% (w/v), or wherein said liquifying agent comprises a DNase that is capable of cleaving DNA in said biological sample.
